# EUROPEAN PATENT SPECIFICATION

(11) **EP 1 321 468 B1**
(45) Date of publication and mention of the grant of the patent: **09.02.2005**
(21) Application number: 02028414.7
(22) Date of filing: 18.12.2002
(51) Int. Cl.: C07F 1/00, C23C 18/42, C07D 233/74, C25D 3/48

(54) **Hydantoin-based gold complex**
Goldkomplex auf der Basis von Hydantoin
Complexe d'or sur base d'hydantoine

(30) Priority: 19.12.2001 JP 2001386147
(43) Date of publication of application: 25.06.2003
(73) Proprietor: Tanaka Kikinzoku Kogyo K.K., Tokyo 103-8206 (JP)
(72) Inventor: Ohtani, Yutaka, Tanaka Kikinzoku Kogyo K. K., Hiratsuka-shi, Kanagawa 254-0076 (JP); Sasaki, Haruko, Tanaka Kikinzoku Kogyo K. K., Hiratsuka-shi, Kanagawa 254-0021 (JP)
(74) Representative: Klunker . Schmitt-Nilson . Hirsch

(56) References cited:
- JP-A- 2000 355 792
- US-A- 3 018 232
- US-B1- 6 511 589

## Description

### BACKGROUND OF THE INVENTION

### 1. FIELD OF THE INVENTION

The present invention relates to a gold complex that is a material for electroless and electrolytic plating solutions used in gold plating to wafers, substrates, and the like.

### 2. DESCRIPTION OF THE RELATED ART

Heretofore, as plating solutions used in electroless and electrolytic plating, cyan-containing gold plating solutions using cyanized gold complexes, which excel in stability in the plating solution as a gold source, have been used. However, since cyan-containing gold salts have a strong toxicity, there are problems from the point of view of labor safety and waste treatment. The use of cyan-containing gold plating solutions also has a problem that excessive cyanides peel or damage the resist patterns of semiconductor parts, thereby making formation of fine circuit patterns difficult.

In view of such problems, the application of plating solutions using gold salts or gold complexes containing no cyan is considered desirable, and for example, cyan-free gold plating solutions, such as a solution of gold sulfite salt (Na₃Au(SO₃)₂) has been proposed.

However, there is another problem that gold salts or gold complexes contained in known cyan-free gold plating solutions have poor stability, and are decomposed during plating operations. For example, in the above-described gold sulfite salt, sulfite ions in the solution may be oxidized by atmospheric oxygen, its concentration decreases and the stability of the gold complex may lower, resulting in the decomposition of the plating solution. When such decomposition occurs, gold-precipitation phenomenon wherein gold in the plating solution precipitates and deposits on the walls of plating tanks or pipes occurs, and interferes with plating operations. Therefore, additives, such as a stabilizer and a complexing agent, are added to the cyan-free gold plating solutions to prevent the decomposition of the plating solution for plating treatment. However, since such measures increase the cost of the stabilizer and make the step of preparing the plating solution complicated, the costs will be elevated.

In addition, the plating solution containing gold salts or gold complexes of poor stability has a problem in the point of view of storage thereof. In the case of the above-described gold sulfite salts, since black precipitations are easily produced due to the decomposition of the gold salts during the storage, the storage in a light-shielded place is essential, and the control thereof is not easy.

The present invention is devised in the above-described background, and the object of the present invention is to provide a cyan-free gold complex that can be applied as a gold source in electroless and electrolytic plating solutions, and has a high stability.

### SUMMARY OF THE INVENTION

In order to solve the above-described problems, the inventors of the present invention carried out repeated examinations, and found a gold complex making a hydantoin compound the ligand by a predetermined operation, as a highly stable gold complex even in a solvent.

Namely, the present invention is a gold complex which can be used as a material for electroless or electrolytic plating solutions and which has been prepared prior to its use, the gold complex having been prepared through allowing a gold hydroxide salt to react with a hydantoin-based compound in an aqueous solution at a temperature between 30 C to 80 C, in order to coordinate the hydantoin-based compound to gold ions.

Combining a gold salt with a hydantoin compound as a chelating agent in an electrolytic plating solution has been described in JP 2000 355792 but this plating solution is not heated prior to electrolysis.

The reason why "allowing a gold hydroxide salt to react with a hydantoin-based compound in an aqueous solution at a temperature between 30 C to 80 C" as described in claim 1 is that gold ions do not bond to the hydantoin compund unless a gold hydroxide salt is mixed with the hydantoin compund in an aqueous solution, and the aqueous solution is heated to such temperature range. in other words, it is because gold ions do not bond to the hydantoin only by mixing the gold hydroxide salt with the hydantoin compound. When simply mixing, although the hydantoin compound in the solution functions as a chelating agent, gold remains in the state of a gold hydroxide complex (Au(OH₄)⁻). Although gold plating can be performed using such a solution, the depositing mechanism is different from the case of the gold complex according to the present invention. The reaction temperature is particularly preferably 60°C, and the reaction time is 30 to 90 minutes, preferably 60 minutes or longer.

In the present invention, the reaction ratio of the gold hydroxide complex to the hydantoin compound for coordinating the hydantoin compound to gold ions is preferably 1:2 to 1:4 in mole ratio. If the mole ratio is 1:2 or below, the stability of the formed gold complex may be poor, and decomposition may occur. If the mole ratio is 1:4 or above, the stability of the formed gold complex is not improved, and in addition, the salting out of the hydantoin compound occurs in the aqueous solution. It is particularly preferable that the mole ratio in this reaction is 1:3.

Regarding to gold salts and hydantoin compounds used as materials for the gold complex according to the present invention, sodium gold hydroxide and potassium gold hydroxide can be used as the gold salt. As the hydantoin compound, hydantoin, 1-methylhydantoin, 3-methylhydantoin, 5-methylhydantoin, 1,3-dimethylhydantoin, 5,5-dimethylhydantoin, 5-metylhydantoinacetic acid, hydantoic acid, and the like can be used. Of these compounds, the coordination of 5,5-dimethylhydantoin imparts especially high stability to the complex after reaction.

The above-described gold complex according to the present invention excels in stability, and the form thereof can be maintained for a long period of time without being decomposed. When the gold complex is applied as the gold source of the plating solution, stable plating operation can be carried out without causing the deposition of gold during the plating operation. The stability of the gold complex according to the present invention is maintained at a high temperature of about 100°C and under direct sunlight. Therefore, the storage of the gold complex according to the present invention is relatively easy.

Since the gold complex of the present invention is formed by a reaction in an aqueous solution, when the complex is used in a plating solution or the like, the solution after the reaction can be used as it is as the material for the plating solution, and by adding additives such as a buffering agent and a heavy metal (thallium etc.) or the like for promoting deposition, the plating solution can be prepared. Also when the utilization of the aqueous solution after the reaction is considered, it is preferable that the pH thereof is adjusted to 7 to 13. Although the complex in the solution will not be decomposed if the pH is less than 7, there is the possibility of hueing when the solution is stored for a long time.

### DETAILED DESCRIPTION OF THE PREFERRED EMBODIMENTS

The preferred embodiments of the present invention will be described below.

### Embodiment1

In 200 mL of water, 60 g of dimethylhydantoin was mixed and dissolved at 60°C. In this aqueous solution of dimethylhydantoin, 1 g of activated charcoal was added. The aqueous solution was heated and stirred for 60 minutes at 60°C, and suction-filtered. Then, a solution prepared by dissolving the quantity equivalent to 30 g of gold of sodium gold hydroxide (Na (Au (OH₄)) was mixed in this aqueous solution of dimethylhydantoin, and heated and stirred for 60 minutes at 60°C to allow them to react. The solution after reaction was cooled to room temperature, the volume of the solution was adjusted to 1 L, and the pH was adjusted to 6 to 9 with the use of phosphoric acid for the following stability test.

### Embodiment 2

The quantity of dimethylhydantoin dissolved in the aqueous solution of dimethylhydantoin of Embodiment 1 was changed to 90g, and the gold hydroxide complex solution same as in Embodiment 1 was mixed and allowed to react. The reaction conditions were the same as in Embodiment 1.

### Embodiment 3

The quantity of dimethylhydantoin dissolved in the aqueous solution of dimethylhydantoin of Embodiment 1 was changed to 120g, and the gold hydroxide complex solution same as in Embodiment 1 was mixed and allowed to react. The reaction conditions were the same as in Embodiment 1.

### Comparative Example 1

In order to compare with Embodiments 1 to 3, a conventional sodium gold sulfite solution was prepared. In this Comparative Example, the quantity equivalent to 40 g of gold of sodium gold sulfite was dissolved in 1 L of water.

The stabilities of the gold complex solution according to Embodiments 1 to 3 and the gold complex solution according to the Comparative Example were examined. The stabilities were evaluated at two temperatures, specifically a high temperature (90°C) and room temperature.

As the evaluation of stability at high temperature, 40 mL of each solution was put in a sample bottle and heated at 90°C for 9 hours, and the hue after heating and the occurrence of precipitation were checked. As a result, no precipitations occurred in solutions according to Embodiments 1 to 3 in any concentration and pH; however, fine reddish brown particles were dispersed in the solution according to Comparative Example after heating. These fine reddish brown particles were considered to be gold compounds formed by the decomposition of the complex.

Next, evaluation at normal temperature was carried out. In this evaluation, each solution was put in a reagent bottle for storage, and placed in the room in the vicinity of window exposed to direct sunlight, in the room not exposed to direct sunlight, and in a cash box (dark place) maintained at 15°C, and the hue of the solutions and the occurrence of precipitation after 1 month of storage were checked. As a result, no precipitations occurred in solutions according to any of Embodiments 1 to 3; however, a salting-out product was observed on the cap of the sample bottle containing the solution according to Comparative Example after storing under direct sunlight.

### Embodiment 4

Next, with regard to the aqueous solution of the gold complex, the oxidation resistance test was performed in an oxidizing atmosphere to examine the stability thereof. First, as in Embodiment 1, an aqueous solution of dimethylhydantoin (concentration: 90 g/L) was mixed with a solution of gold hydroxide complex (gold content: 15 g/L), and allowed to react in the same manner as Embodiment 1. In this reacting solution, 50 g/L of trisodium phosphate (Na₃PO₄·12H₂O) and 30 g/L of sodium dihydrogen phosphate (NaH₂PO₄) were added as buffering agents, and this solution was used as the test solution.

### Comparative Example 2

To compare with Example 4, 50 g/L of an aqueous solution of sodium sulfite was added to a conventional aqueous solution of sodium gold sulfite (gold content: 10 g/L), and this solution was used as the comparative test solution.

The oxidation resistance of test solutions according to Embodiment 4 and Comparative Example 2 was examined. The evaluation of oxidation resistance was conducted by way of putting 30 mL of each plating solution in a sample bottle, adding 3 mL of a hydrogen peroxide solution (34.5%) to each bottle, stirring each solution and observing the formation of precipitations after being left for 9 hours and 50 hours.

As a result, in the plating solution of Comparative Example 2, although no change was observed immediately after the addition of the hydrogen peroxide solution, the occurrence of fine particles was observed 9 hours later, and the deposition of gold was observed on the wall of the sample bottle 50 hours later. It is considered the phenomenon happened because the sulfite ions in gold sulfite complex were oxidized by the addition of hydrogen peroxide to form sulfuric acid. On the other hand, such a change was not observed in the plating solution according to Embodiment 4. From this fact, it was verified that the plating solution according to Embodiment 4 excelled in oxidation resistance.

### Embodiment 5

Here, plating was performed with the use of a plating solution prepared from the gold complex solution according to Embodiment 2, and the characteristics of the film were examined. The plating solution was prepared through mixing the gold complex solution prepared in Embodiment 2, trisodium phosphate, and sodium dihydrogen phosphate. The composition of the plating solution and the conditions of plating were as follows:

| Composition of plating solution | |
|---|---|
| Gold complex solution | 15 g/L (as gold) |
| Trisodium phosphate | 50 g/L |
| Sodium dihydrogen phosphate | 30 g/L |
| Crystal regulator | as required |

| Plating conditions | |
|---|---|
| Substrate | Silicon wafer |
| Solution temperature | 50°C |
| pH | 7 |
| Current density | 1.5 A/dm ² |

Under the above conditions, a bump of a size of 40 × 80 µm, and a thickness of 20 µm was formed on a substrate. Immediately after plating, the Vickers hardness of the bump was 90 to 110 Hv. The hardness of the bump when the substrate after plating was heat-treated at 300°C for 30 minutes in a nitrogen atmosphere was 40 to 70 Hv. The hardness of this bump was substantially equal to the hardness of the bump formed using a conventional gold sulfite salt.

As described above, the gold complex according to the present invention excels in stability, and the form can be maintained for a long period of time without being decomposed. The gold complex according to the present invention can maintain stability even at a high temperature and under direct sun light, and the storage thereof is easy. The gold complex according to the present invention is suitable as a gold source for electroless and electrolytic plating solutions, and enables stable plating operations without depositing gold during plating operations.

## Claims

1. A gold complex which can be used as a material for electroless or electrolytic plating solutions and which has been prepared prior to its use, the gold complex having been prepared through allowing a gold hydroxide salt to react with a hydantoin-based compound in an aqueous solution at a temperature between 30 C to 80 C, in order to coordinate the hydantoin-based compound to gold ions.

2. The gold complex according to claim 1, wherein the reaction ratio of the gold hydroxide salt to the hydantoin-based compound is 1:2 to 1:4 in mole ratio.

3. The gold complex according to claim 1 or 2, wherein the gold hydroxide salt is sodium gold hydroxide or potassium gold hydroxide.

4. The gold complex according to any of claims 1 to 3, wherein the hydantoin-based compound is 5,5-dimethylhydantoin.

## Patentansprüche

1. Goldkomplex, welcher als Material für Lösungen zur nicht-elektrischen oder elektrolytischen Beschichtung verwendet werden kann und vor seiner Verwendung hergestellt worden ist, wobei der Goldkomplex hergestellt worden ist durch Reagierenlassen eines Goldhydroxidsalzes mit einer Hydantoin-basierten Verbindung in einer wässrigen Lösung bei einer Temperatur zwischen 30 °C bis 80 °C, um die Hydantoin-basierte Verbindung an die Goldionen zu koordinieren.

2. Goldkomplex nach Anspruch 1, bei welchem das Reaktionsverhältnis des Goldhydroxidsalzes zu der Hydantoin-basierten Verbindung 1:2 bis 1:4 im molaren Verhältnis ist.

3. Goldkomplex nach Anspruch 1 oder 2, , bei welchem das Goldhydroxidsalz Natriumgoldhydroxid oder Kaliumgoldhydroxid ist.

4. Goldkomplex nach einem der Ansprüche 1 bis 3, bei welchem die Hydantoin-basierte Verbindung 5,5-Dimethylhydantoin ist.

## Revendications

1. Complexe d'or pouvant être utilisé en tant que matière première pour des solutions de métallisation auto-catalytique et électrolytique et préparé avant son utilisation, le complexe d'or ayant été préparé par réaction d'un sel d'hydroxyde d'or avec un composé à base d'hydantoïne dans une solution aqueuse à une température entre 30°C et 80°C afin de coordiner le composé à base d'hydantoïne aux ions d'or.

2. Complexe d'or selon la revendication 1, le rapport de réaction du sel d'hydroxyde d'or au composé à base d'hydantoïne étant de 1:2 à 1:4, en rapport molaire.

3. Complexe d'or selon la revendication 1 ou 2, le sel d'hydroxyde d'or étant l'hydroxyde d'or et de sodium ou l'hydroxyde d'or et de potassium.

4. Complexe d'or selon l'une quelconque des revendications 1 à 3, le composé à base d'hydantoïne étant la 5,5-diméthylhydantoïne.
